# EUROPEAN PATENT APPLICATION

(11) **EP 0 544 413 A1**
(43) Date of publication of application: **02.06.1993**
(21) Application number: 92309985.7
(22) Date of filing: 30.10.1992
(51) Int. Cl.: C12Q 1/18

(54) **Testing animal fluids**

(30) Priority: 30.10.1991 GB 9123062
(71) Applicant: TEPNEL MEDICAL LIMITED, Manchester M7 4LE (GB)
(72) Inventor: Wahbi, Laith, Manchester, M21 2TL (GB); Brown, Allan, Flat 4, Manchester, M21 1SN (GB); Hood, Robert, Paisley, PA2 6HW (GB); Minter, Stephen John, New Mills, SK12 4QL (GB); Musialowski, Michael, c/o Tepnel Medical Limited, Manchester, M1 4LF (GB)
(74) Representative: Atkinson, Peter Birch

(57) **Abstract**

A method for testing animal fluids such as milk or blood for the presence of antimicrobial agents. The method relies upon testing for growth in the fluid of an indicator organism which is resistant to the antimicrobial agent. The fluid may be tested for the presence of a range of antimicrobial agents by testing for growth in the fluid of a series of indicator organisms which are selectively resistant to one or more antimicrobial agents. The indicator organism may be one which has been produced by transformation (using genetic engineering techniques) of an organism which is sensitive to the antimicrobial agent. The sensitive organism may be transformed using a plasmid vector into which a gene providing the desired antimicrobial resistance has been cloned. If the sensitive organism is required to be resistant to two or more antimicrobial agents then the organism may be transformed with two or more genes, as required. The introduction of the antimicrobial resistance factor should affect only the host's sensitivity to the corresponding antimicrobial agent or cross-functional class of agents and not the host phenotype with respect to the rest of its antimicrobial sensitivity spectrum.

## Description

This invention relates to testing animal fluids - such, primarily, as milk, but also blood as an indicator for animal meat - for the presence of antimicrobial agents such as antibiotics which are administered to animals.

It is desirable that animal product for human consumption be free of such antibiotics in order to avoid building up in the population resistant strains of the organisms against which the antibiotics are specific, thereby rendering the antibiotics generally ineffective.

To this end, several screening systems have been devised for the routine testing of milk. While collectively the various systems will detect the presence of most antibiotics currently in use, the individual systems each cover only some of those. The systems moreover are inconvenient and slow in operation, so that contamination of a batch of milk may not be detected until after the milk has been mixed with other milk or distributed or processed, which necessitates wasting incontaminated milk, recalling already distributed milk and even the closure for purging of processing plant.

The present invention provides a system which is convenient in operation, which requires only compact and portable apparatus, which is sensitive to as broad a range of antibiotics as may be desired, and which is fast in operation.

The invention comprises a method for testing animal fluids for the presence of antimicrobial agents comprising testing for growth in the fluid of an indicator organism which is resistant to the antimicrobial agent.

The term animal fluid as used herein includes any fluid which is produced by, present in or derived from, an animal. Examples of such fluids are milk and blood.

In its broadest concept, therefore, the invention provides for testing for the presence of a single antimicrobial agent. The invention is however particularly suitable for testing the fluid for the presence of a range of antimicrobial agents by testing for growth in the fluid of a series of indicator organisms which are selectively resistant to one or more antimicrobial agents.

If desired, the organism may be resistant to two or more antimicrobial agents.

The indicator organism may be one which has been produced by transformation (using genetic engineering techniques) of an organism which is sensitive to the antimicrobial agent. The sensitive organism may be transformed using a plasmid vector into which a gene providing the desired antimicrobial resistance has been cloned. If the sensitive organism is required to be resistant to two or more antimicrobial agents then the organism may be transformed with two or more genes, as required.

The introduction of the antimicrobial resistance factor should affect only the host's sensitivity to the corresponding antimicrobial agent or cross-functional class of agents and not the host phenotype with respect to the rest of its antimicrobial sensitivity spectrum.

The sensitive organism may be Bacillus megaterium which is particularly suitable because it is sensitive to a broad range of antibiotics. Thus by producing genetically engineered forms of the organism resistant to a range of antimicrobial agents, the invention is able to provide an extremely sensitive test for a range of antimicrobial agents. Furthermore Bacillus megaterium is a spore former which allows freeze drying of the transformer organisms which thus provides the organisms in a convenient form for storage prior to being used in the test. Other organisms may also be used, e.g. E. coli.

If the method of the invention is carried out to detect the presence or absence of a range of antimicrobial agents then the series of organisms (each resistant to a particular antimicrobial agent or combination of antimicrobial agents) is preferably an isogenic series of organisms, e.g. an isogenic series of Bacillus megaterium.

The gene which is incorporated into the organism may be one which provides resistance to any desired antimicrobial agent, e.g. Penicillin G(IU), Cephalexin, Kanamycin, Neomycin, Streptomycin, Erythromycin, Tetracycline, Bacitracin, Polymyxin, Chloramphenicol, Lincomycin, Trimethoprim, Sulphamethoxazole, Sulphathiazine, or Novobiocin. Details of genes required for providing antimicrobial resistance are available in the literature, see for example Molecular Biological Method for Bacillus (pages 75-556), ed. C.R. Harwood & S.M. Culting, John Wiley & Sons Chichester; Mol Gen Genet (1990) 223, pages 185-191 Peter Haima et al; and, Gene 86 (1990) pages 63-69, Elsevier, Peter Haima et al.

The genes may be incorporated into the organism using known plasmid vectors. For example, a gene providing resistance to Chloramphenicol may be introduced using the plasmid pHV33 whereas a gene providing resistance to Tetracycline may be introduced using the plasmid pT181.

The method of the invention may if desired (or required) be conducted in conjunction with a control experiment which utilises the sensitive organism.

The individual strains of the organisms may be provided in inactive form in culture chambers into which the fluid is introduced for the test. The organisms may be freeze dried together with nutrients and adjuvants as necessary into the chambers. The chambers may be wells of a microtitre tray.

Growth may be tested for optically, as by a colour change. For example, the fluid being tested may incorporate a redox indicator which changes colour as the redox potential of the fluid reduces due to growth of the organism and reduction of nutrients. Rezarin is a particularly suitable redox initiator and changes colour from purple to pink upon reduction of the redox potential. The fluid may be illuminated and light from the fluid sensed photoelectrically. It is particularly preferred that the light which is sensed is transmitted, rather than reflected, light. On the other hand a luminescent organism may be used, in which case growth of the organism (which indicates presence of the antimicrobial agent) is sensed by the luminescence produced without the need to illuminate the fluid. A luminescent organism can of course be produced by transforming the organism with a gene which expresses luciferase.

It is preferably arranged that detectable growth occurs within one hour - thirty minutes is possible - unless growth is inhibited by an agent.

The method may be applied to the testing of milk, where its broad spectrum of detection and ease and speed of operation bring substantial advantages over presently available systems. The method may also be applied to blood, e.g. from an animal before slaughter - a positive test may indicate deferral of slaughter until the animal tests clear.

The invention also comprises apparatus for testing animal fluids for the presence of an antimicrobial agent comprising at least one culture chamber containing an indicator organism (and necessary nutrients and adjuvants) into which the fluid can be introduced, the organism being resistant to the agent.

One embodiment of apparatus in accordance with the invention comprises a plurality of culture chambers each incorporating the same indicator organism, and possibly also at least one further chamber incorporating a "control" organism. Such an apparatus is useful for testing animal fluids from a number of different animals for the presence of the same antimicrobial agent.

A further embodiment of apparatus in accordance with the invention comprises a plurality of culture chambers each incorporating different indicator organisms selectively resistant to one or more antimicrobial agents, and possibly also at least one further chamber incorporating a "control" organism. This embodiment of apparatus is useful for testing a fluid from a particular animal for the presence of a range of antimicrobial agents.

The organisms may be freeze dried in the chambers, which may be wells of a microtitre tray.

The apparatus may comprise temperature control means, and also a shaker, for the chambers.

The apparatus may comprises optical detection means for detecting growth in the chambers. The chambers may be illuminated and photo-electric sensor means arranged to detect light from the chambers. The chambers may be illuminated with monochromatic light (as by a lamp shining through a filter at e.g. 600nm) and the detector means detect a colour change due to growth. The organism may on the other hand be luminescent and photo-electric sensor means arranged to detect light from the organism to indicate growth.

Light from the chambers may be conveyed to an array of photo-electric detectors by optical fibres. However, a single photo-electric detector may be arranged to scan the chambers.

The apparatus may also comprise information processing means receiving growth information from the chambers and programmed to report on antimicrobial agents detected in a test.

Embodiments of apparatus and methods for testing animal fluids for the presence of antimicrobial agents will now be described with reference to the accompanying drawings in which :-
Figure 1 is a diagrammatic illustration of a single culture chamber and optical detection means;
Figure 2 is a diagrammatic illustration of a first embodiment of apparatus, and
Figure 3 is a diagrammatic illustration of a second embodiment of apparatus.

The drawings illustrate methods and apparatus for testing animal fluids such as milk 11 for the presence of antimicrobial agents comprising testing for growth in the fluid 11 of indicator organism 12 selectively resistant to one or more agents.

One series of organisms found particularly advantageous is an isogenic series of Bacillus megaterium variants, which has been generate by using plasmid vectors into which antibiotic resistant genes or combinations of genes have been cloned. This micro-organism exhibits a greater broad-spectrum sensitivity to a range of veterinary antimicrobial agents than any other commonly used mesophilic indicator organism. The introduction of an antibiotic resistance factor affects only the host's sensitivity to the corresponding antibiotic agent or cross-functional class of agents.

It does not change the host phenotype with respect to the rest of its antibiotic/antimicrobial sensitivity spectrum.

A single test using B.megaterium with no resistant gene would of course disclose the presence of any one or more antibiotics, but would not enable any identification. Growth of B.megaterium having a single gene specific to resistance to one antibiotic indicates that either no antibiotic is present or only the specified antibiotic is present; to decide if that particular antibiotic is present it is necessary to see if a strain not resistant to it fails to grow. Commonly available antibiotic preparations may contain two or more agents. B.megaterium can be prepared with two or more genes imparting resistance to all the agents in a particular preparation.

Thus, if an array of culture chambers are each innoculated with indicator organisms resistant to different antimicrobial agents or combinations of agents (each organism possessing a unique phenotype) some chambers will exhibit growth upon introduction of a contaminated sample and others will not. A pattern of growth can be observed which will be unique to an agent or combination of agents which will enable particular preparations to be identified.

The organisms 12 in inactive state are provided in culture chambers 13; the organisms may be freeze dried and supplied with nutrients and adjuvants as necessary. For example, chamber 13 may include an oxoid media such as 1S0-S. The fluid 11 is simply added for the test, the chamber warmed to an optimal growth temperature, e.g. 30°C, and shaken. Because the test is carried out in liquid suspension growth is rapid, and detectable growth occurs under these conditions within 30 minutes - it is desirable that growth should be detectable within a short period of time such as 30 minutes or an hour where perishable produce such as milk is concerned. It is perhaps less important, but still desirable, for the test to give a rapid result when testing an animal before slaughter, by testing its blood to see if the meat will be contamined by antibiotics, but of course such a test could be carried out on blood recovered after slaughter, in which case a rapid result would be important to divert the carcase out of the supply line.

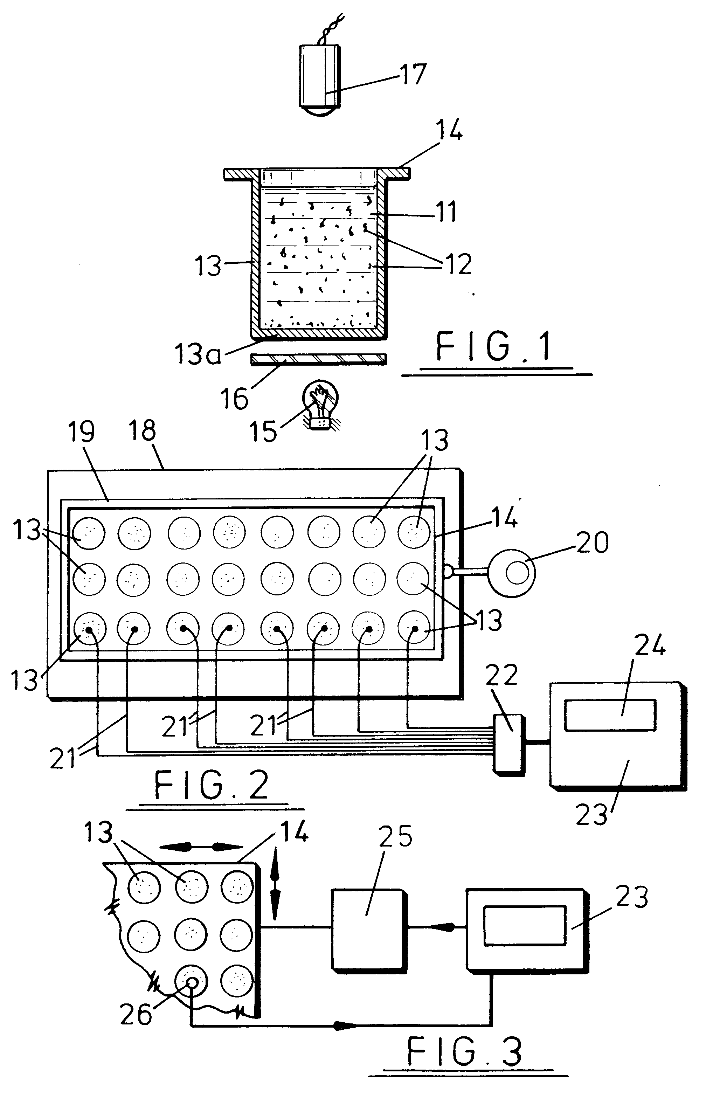

The chamber 13 of Figure 1 can be a well of a microtitre plate 14 such as is shown in Figures 2 and 3. The microtitre plate may for example be of polycarbonate or polystyrene. Chamber 13 has a light transmissive base 13a through which the contents of the chamber are illuminated by a filament lamp 15 shining through a monochromatic filter 16 passing say 600 nm light. The light transmitted through the fluid in the chamber is detected by a photoelectric device 17. As indicated above, the chamber 13 may incorporate a redox indicator (e.g. Rezarin) which changes colour upon growth of the organism.

Figure 2 illustrates an arrangement in which a section of microtitre plate 14 has a 3 x 8 array of wells 13. It is of course possible to use other array sizes, e.g. 8 x 8. Each well is assumed to contain a different indicator organism thereby permitting a range of antimicrobial agents to be detected. The plate 14 is contained in an incubator 18 on a Peltier heating block 19 with thermostatic control and has a shaker mechanism 20.

The apparatus includes a detector arrangement which incorporates eight optical fibres 21 which guide detected light to an array 22 of photodiodes, the outputs of which are connected to a computer 23.

In use of the apparatus the eight optical fibres each detect light from a respective one of eight chambers of the microtitre tray 14. The detected light from each chamber is recorded by computer 23. The microtitre tray 14 is then indexed relative to the optical fibres 21 to present a further row of eight chambers 13 for detection.

It will be appreciated that computer 23 will have a record of the antimicrobial agent which organisms in each of the chambers 13 are adapted to detect. The computer 23 is programmed to compare this recorded information with the results obtained for the particular fluid sample under test (i.e. the chambers in which growth of the organism was found to occur) and provide a display (e.g. on a screen 24) or a print-out of the antimicrobial agents which have been detected in the sample.

It should also be appreciated that each fluid sample to be tested may be supplied in a bottle or other vessel labelled with a bar-code and that a corresponding bar-code may be provided on the microtitre tray into which aliquots of the fluids are introduced. A bar-code reader (not shown) will then be provided in the apparatus of Fig.2 for reading the bar-code on the microtitre tray. The use of such a bar-code system facilitate correlation of the results of the test with the original fluid sample.

Figure 3 illustrates an arrangement in which the plate 14 is moved by a motor mechanism 25 to present each well 13 in turn to a photodiode 26 inputting serially to the alalyzer 20, which controls the mechanism 25 to scan the wells to carry out the test.

In a modification of the above described arrangements, it is possible to test for organism growth in only selected ones of the chambers 13 if it is desired to test for only a restricted number of antimicrobial agents.

In a further modification of the above described arrangements, the wells of the microtitre tray may include the same indicator organism whereby a number of fluids from different animals may be tested for the presence of the same antimicrobial agent.

## Claims

1. A method for testing animal fluids for the presence of antimicrobial agents comprising testing for growth in the fluid of an indicator organism which is resistant to the antimicrobial agent.

2. A method as claimed in claim 1 which comprises testing for growth in the fluid of a series of indicator organisms which are selectively resistant to one or more agents.

3. A method as claimed in claim 1 or 2 wherein the organism is Bacillus megaterium.

4. A method as claimed in claim 2 wherein the organism is an isogenic series of Bacillus megaterium.

5. A method as claimed in any one of claims 1 to 4 wherein the or each organism has been generated by using a plasmid vector into which an antimicrobial resistant gene or combination of genes has been cloned.

6. A method as claimed in any one of claims 1 to 5 wherein the or each organism is provided in inactive form in a culture chamber into which the fluid is introduced for the test.

7. A method as claimed in claim 6 in which the organism are freeze-dried together with nutrient and adjuvants as necessary into the chambers.

8. A method as claimed in claim 6 or 7 wherein the chambers are wells of a microtitre tray.

9. A method as claimed in any one of claims 1 to 8 wherein growth is tested for optically.

10. A method as claimed in claim 9 wherein a colour change is detected.

11. A method as claimed in claim 10 wherein the colour change is provided by a redox indicator system.

12. A method as claimed in claim 11 wherein the indicator system is Rezarin.

13. A method as claimed in any one of claims 7 to 12 in which the fluid is illuminated and light from the fluid is sensed photoelectrically.

14. A method according to claim 9 wherein the organism is luminescent.

15. A method as claimed in any one of claims 1 to 14 in which detectably growth occurs within one hour unless growth is inhibited by an antimicrobial agent.

16. A method as claimed in any one of claims 1 to 15 applied to the testing of milk.

17. A methods as claimed in any one of claims 1 to 15 applied to the testing of blood.

18. Apparatus for testing animal fluids for the presence of an antimicrobial agent comprising at least one culture chamber containing an indicator organism (and necessary nutrients and adjuvants) into which the fluid can be introduced, the organism being resistant to the agent.

19. Apparatus as claimed in claim 18 comprising a plurality of said culture chambers which contain a series of indicator organisms which are selectively resistant to one or more agents.

20. Apparatus as claimed in claim 18 comprising a plurality of cambers incorporating organisms which are resistant to the same antimicrobial agent or combination of antimicrobial agents.

21. Apparatus as claimed in claim 19 or 20 wherein the culture chambers are wells of a microtitre tray.

22. Apparatus according to any one of claims 18 to 21 in which the organisms are freeze-dried.

23. Apparatus according to any one of claims 18 to 22 comprising temperature control means for the chambers.

24. Apparatus as claimed in any one of claims 18 to 23 comprising optical detection means for detecting growth in the chambers.

25. Apparatus as claimed in claim 24 in which the chambers are illuminated and photoelectric sensor means are arranged to detect light from the chambers.

26. Apparatus according to claim 25 wherein the chambers are illuminated with monochromatic light and the detector means detects a colour change due to growth.

27. Apparatus according to claim 24 wherein the organism is luminescent and photoelectric sensor means are arranged to detect light from the organism to indicate growth.

28. Apparatus as claimed in any one of claims 24 to 27 wherein light from the chambers is passed to an array of photoelectric detectors by optical fibres.

29. Apparatus as claimed in any one of claims 24 to 27 wherein a single photoelectric detector is arranged to scan the chambers.

30. Apparatus as claimed in any one of claims 18 to 29 comprising information processing means receiving growth information from the chambers and programmed to report on antimicrobial agents detected in a test.
